# EUROPEAN PATENT SPECIFICATION

(11) **EP 3 388 055 B1**
(45) Date of publication and mention of the grant of the patent: **17.11.2021**
(21) Application number: 16872387.2
(22) Date of filing: 07.12.2016
(51) Int. Cl.: A61K 9/127, A61K 31/4745, A61K 31/337, A61K 31/436, A61K 31/5517, A61P 35/00, A61P 35/02

(54) **METHOD FOR PREPARING LIPOSOME**
VERFAHREN ZUR HERSTELLUNG VON LIPOSOMEN
PROCÉDÉ DE PRÉPARATION D'UN LIPOSOME

(30) Priority: 08.12.2015 CN 201510897554
(43) Date of publication of application: 17.10.2018
(73) Proprietor: Chia Tai Tianqing Pharmaceutical Group Co., Ltd., Jiangsu Province 222062 (CN)
(72) Inventor: ZHANG, Xiquan, Lianyungang Jiangsu 222062 (CN); DONG, Ping, Lianyungang Jiangsu 222062 (CN); ZHANG, Huanqing, Lianyungang Jiangsu 222062 (CN); CHENG, Yanju, Lianyungang Jiangsu 222062 (CN); ZHOU, Hao, Lianyungang Jiangsu 222062 (CN); JIANG, Bo, Lianyungang Jiangsu 222062 (CN); LIU, Fei, Lianyungang Jiangsu 222062 (CN)
(74) Representative: Brand Murray Fuller LLP
(86) International application number: PCT/CN2016/108840
(87) International publication number: WO 2017/097196

(56) References cited:
- EP-A1- 0 750 910
- WO-A2-2004/071466
- CN-A- 1 753 657
- CN-A- 101 244 039
- CN-A- 101 283 983
- CN-A- 102 516 258
- CN-A- 102 949 343
- US-A1- 2003 100 511
- US-A1- 2005 202 076
- US-A1- 2006 110 441
- CORTESI RITA ET AL: "Ethosomes for the delivery of anti-HSV-1 molecules: Preparation, characterization and in vitro activity", PHARMAZIE, vol. 65, no. 10, October 2010 (2010-10), pages 743-749, XP055597052, DOI: 10.1691/ph.2010.0106.Source:

## Description

### TECHNICAL FIELD

The present application relates to a new method for preparing a liposome and a liposome prepared by this method.

### BACKGROUND

In order to improve the solubility of a poorly water-soluble or water-insoluble drug, a pharmaceutical preparation technique, such as an emulsion, a micelle, a liposome, and the like, is used in the development of a pharmaceutical preparation of such drug. However, emulsion and micelle preparations have some disadvantages. For example, an emulsion is a thermodynamically unstable system that is prone to aggregation, fusion, flocculation, oxidation, degradation, hydrolysis, and so on during the storage process, thereby affecting the quality of the emulsion and the therapeutic efficacy of the drug. For another example, micelle preparations usually utilize a surfactant to form micelles so as to solubilize a drug. However, a surfactant may produce a toxic and side effect in clinical use, trigger a hypersensitive response, and thereby affect the medication safety. A liposome can change the in vivo distribution of a drug, reduce the toxicity of a drug, alleviate an allergic reaction and immune response, and extend the release of a drug. However, a liposomal preparation obtained by existing methods is usually in a liquid state, which is a thermodynamically unstable system, and has the problems of low stability and low entrapment efficiency, easy leakage of a drug, and bacteria breeding, sedimentation and aggregation during the storage process, difficulty in controlling the particle size, and wide particle size distribution. Even after reconstitution into liquid liposomes after freeze drying, it is difficult to be reconstituted, and the reconstituted liposome has a large particle size and a wide particle size distribution.

Chinese patent No. ZL201110355747.5 discloses a compound represented by formula I (also known as moexitecan (Chinese name: 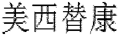 ),

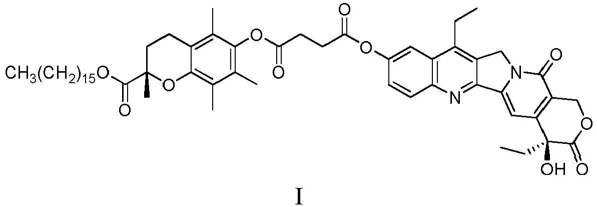

Moexitecan is insoluble or almost insoluble in water and an aqueous medium, which is a water-insoluble drug. This patent discloses that this drug may be formulated into emulsions, microemulsions, or micelles. However, it is found that after formulating into the emulsions, microemulsions, or micelles, these preparations have very poor stability, and the micelle preparation has very high toxicity. Therefore, there is an urgent need to develop a new pharmaceutical preparation suitable for a poorly water-soluble or water-insoluble drug and a preparation method thereof.

### SUMMARY

In an aspect, the present application provides a method for preparing a liposome, comprising:
(1) dissolving a substance to-be-entrapped and a phospholipid in an organic solvent to obtain an organic phase, and then mixing the organic phase with an aqueous phase to obtain a liposome feed liquid;
(2) extruding the liposome feed liquid obtained in step (1) through a polycarbonate membrane; and
(3) lyophilizing,
wherein the phospholipid is a combination of yolk phosphatidylcholine and hydrogenated soybean phosphatidylcholine; and the substance to-be-entrapped is moexitecan.

In another aspect, the present application provides a liposome prepared by the above-mentioned preparation methods, wherein the liposome can be reconstituted after the addition of water or an aqueous solvent and the reconstituted liposome has a particle size of 50-400 nm or 100-250 nm, preferably wherein the liposome has an entrapment efficiency > 99% and a particle size distribution index of below 0.18.

### DETAILED DESCRIPTION OF THE INVENTION

In the following description, certain specific details are included to provide a thorough understanding of various disclosed embodiments. However, those skilled in the relevant art will recognize that the embodiments may be practiced without one or more of these specific details, or with other methods, components, materials, and the like.

Unless the context requires otherwise, throughout the specification and claims which follow, the term "comprise" and English variations thereof, such as "comprises" and "comprising", are to be construed in an open and inclusive sense, that is as, "including, but not limited to".

Reference throughout this specification to "one embodiment", or "an embodiment", or "another embodiment", or "some embodiments" means that a particular referent element, structure, or characteristics described in connection with the embodiment is included in at least one embodiment. Accordingly, the appearances of the phase "in one embodiment", or "in an embodiment", or "in another embodiment", or "in some embodiments" in various places throughout this specification are not necessarily all referring to the same embodiment. In addition, the particular elements, structures, or characteristics may be combined in any suitable manner in one or more embodiments.

It should be noted that, as used in this specification and the appended claims, the singular forms "a", "an" and "the" include plural referents unless the content clearly dictates otherwise. Thus, for example, reference to a reaction in which "a catalyst" is involved includes a single catalyst, or two or more catalysts. Unless otherwise explicitly specified herein, it should also be noted that the term "or" is generally employed in its sense including "and/or" unless the content clearly dictates otherwise.

In an aspect, the present application provides a method for preparing a liposome, comprising:
(1) dissolving a substance to-be-entrapped and a phospholipid in an organic solvent to obtain an organic phase, and then mixing the organic phase with an aqueous phase to obtain a liposome feed liquid;
(2) extruding the liposome feed liquid obtained in step (1) through a polycarbonate membrane; and
(3) lyophilizing,
wherein the phospholipid is a combination of yolk phosphatidylcholine and hydrogenated soybean phosphatidylcholine; and the substance to-be-entrapped is moexitecan.

In some embodiments of the present application, a lyoprotectant may be added to the aqueous phase in step (1) or just before performing the lyophilization in step (3).

In some embodiments of the present application, water for injection is added, and subpackaged just before performing the lyophilization in step (3).

In some embodiments of the present application, water for injection is added, sterilized by filtration, and then subpackaged just before performing the lyophilization in step (3).

Disclosed herein is another method for preparing a liposome, comprising:
(1) dissolving a substance to-be-entrapped and a phospholipid in an organic solvent to obtain an organic phase, and then mixing the organic phase with an aqueous phase to obtain a liposome feed liquid;
(2) extruding the liposome feed liquid obtained in step (1) through a polycarbonate membrane; and
(3) adding water for injection, sterilizing by filtration, subpackaging and lyophilizing;
wherein a lyoprotectant is added to the aqueous phase in step (1) or before performing the sterilization by filtration in step (3).

The substance to-be-entrapped is moexitecan.

The phospholipid is a combination of yolk phosphatidylcholine and hydrogenated soybean phosphatidylcholine. Preferably, the phospholipid is a combination of yolk phosphatidylcholine and hydrogenated soybean phosphatidylcholine at a weight ratio of yolk phosphatidylcholine to hydrogenated soybean phosphatidylcholine of 3:1.

In some embodiments of the present application, the organic solvent is one or more selected from the group consisting of anhydrous ethanol, 95% ethanol, methanol, propanol, trichloromethane, dichloromethane, tert-butanol, n-butanol, acetone, methylpyrrolidone, ethyl acetate, isopropyl ether and diethyl ether. Preferably, the organic solvent is selected from the group consisting of anhydrous ethanol, 95% ethanol and tert-butanol. More preferably, the organic solvent is anhydrous ethanol.

In some embodiments of the present application, a weight ratio of the substance to-be-entrapped to the phospholipid is 1:1-1:500. Preferably, the weight ratio of the substance to-be-entrapped to the phospholipid is 1:1-1:100. More preferably, the weight ratio of the substance to-be-entrapped to the phospholipid is 1:15-1:50. Still more preferably, the weight ratio of the substance to-be-entrapped to the phospholipid is 1:20.

In some embodiments of the present application, a weight ratio of the substance to-be-entrapped to the organic solvent is 1:1-1:100. Preferably, the weight ratio of the substance to-be-entrapped to the organic solvent is 1:9-1:50. More preferably, the weight ratio of the substance to-be-entrapped to the organic solvent is 1:30.

In some embodiments of the present application, the aqueous phase comprises water as a major component or substantially consists of water, such as deionized water, distilled water, purified water, water for injection, and the like, preferably water for injection.

In some embodiments of the present application, the organic phase is mixed with the aqueous phase at a temperature of 25-80°C. Preferably, the organic phase is mixed with the aqueous phase at a temperature of 55-65°C.

In some embodiments of the present application, the organic phase may be mixed with the aqueous phase under the protection of nitrogen gas.

In some embodiments of the present application, a pore size of the polycarbonate membrane is selected from the group consisting of 0.015, 0.03, 0.05, 0.08, 0.1, 0.2, 0.4, 0.6, 0.8, 1.0, 2.0, 3.0, 5.0, 8.0, 10.0, and 12.0 µm, preferably 0.1 µm or 0.2 µm. Optionally, a polyester membrane may be additionally added below the polycarbonate membrane.

In some embodiments of the present application, the extrudation may be carried out in any manner, as long as a liposome having a large particle size can become one having a small particle size after passing through the membrane. The temperature of the feed liquid in this step needs to be controlled at 25 °C -80°C, preferably 55 °C -65°C.

In some embodiments of the present application, the lyoprotectant is one or more selected from the group consisting of mannitol, glucose, galactose, sucrose, lactose, maltose and mycose. Preferably, the lyoprotectant is one or more selected from the group consisting of sucrose, mycose and mannitol. More preferably, the lyoprotectant is selected from sucrose or a combination of sucrose and mannitol. Still more preferably, the lyoprotectant is selected from sucrose or a combination of sucrose and mannitol at a weight ratio of sucrose to mannitol of 2:1.

Optionally, in some embodiments of the present application, an antioxidant may be further added to the organic phase in step (1). The antioxidant is one or more selected from the group consisting of sodium sulfite, sodium bisulfite, sodium pyrosulfite, sodium thiosulfate, vitamin C, ascorbyl palmitate, tert-butyl-4-hydroxyanisole (BHA), di-tert-butyl-4-hydroxytoluene (BHT), vitamin E acetate, cysteine and methionine. Preferably, the antioxidant is selected from the group consisting of sodium pyrosulfite, tert-butyl-4-hydroxyanisole, di-tert-butyl-4-hydroxytoluene and vitamin E acetate. More preferably, the antioxidant is selected from di-tert-butyl-4-hydroxytoluene or sodium pyrosulfite.

Optionally, in some embodiments of the present application, the aqueous phase in step (1) may further comprise a metal ion chelating agent. The metal ion chelating agent is selected from the group consisting of disodium edetate, sodium calcium edetate, 1,2-diaminocyclohexane tetraacetic acid, diethylenetriamine pentaacetic acid, trisodium N-(2-hydroxyethyl)-ethylenediamine triacetate and N-di(2-hydroxyethyl)glycine. Preferably, the metal ion chelating agent is selected from disodium edetate or sodium calcium edetate.

Optionally, in some embodiments of the present application, a pH regulator may be further added before performing the lyophilization in step (3) or after adding the lyoprotectant in step (3). The pH regulator is selected from the group consisting of hydrochloric acid, sulfuric acid, acetic acid, phosphoric acid, citric acid, tartaric acid, maleic acid, sodium hydroxide, sodium bicarbonate, disodium hydrogen phosphate, sodium dihydrogen phosphate and sodium citrate. Preferably, the pH regulator is selected from hydrochloric acid or sodium hydroxide. In some embodiments of the present application, the pH is adjusted in a range of 2-10, preferably 4-7.

In some embodiments of the present application, the liposome obtained by the preparation methods according to the present application can be rapidly reconstituted after the addition of water or an aqueous solvent, and the reconstituted liposome has a particle size of 50-400nm, preferably 100-250nm. In some embodiments of the present application, the particle size distribution index is 0.5 or smaller, preferably 0.23 or smaller.

The preparation methods according to the present application have one or more of the following advantages: (1) the preparation process is simple, only requires the steps of dissolving, keeping at a constant temperature, mixing, extruding, lyophilizing and so on, and is particularly suitable for large-scale industrial production; (2) the liposome feed liquid before lyophilization is sterilized by filtration through a 0.22 µm membrane, then aseptically filled and lyophilized, which can be easily achieved in industrial production, and ensure that the product is sterile; (3) the lyophilized liposome has a good stability, and is not significantly changed in key quality index(es), such as particle size, content, related substance(s), entrapment efficiency, or the like, compared with that at the 0th month; and (4) the lyophilized product is almost free of residual solvent.

Compared with a liposome prepared by conventional methods in the art, a liposome obtained by the preparation methods according to the present application has one or more of the following advantages: (1) the liposome according to the present application has high entrapment efficiency (>99%), no leakage of a drug and no decrease in entrapment efficiency during the storage process; (2) the liposome according to the present application has very narrow particle size distribution after extrusion through the polycarbonate membrane several times, and a distribution index of below 0.18, and thereby the particle size and particle size distribution of the liposome are well controlled; (3) compared with ordinary pharmaceutical preparations (e.g., a micelle preparation or an emulsion), the liposome according to the present application has been proved by animal experiments to have reduced the toxicity of the pharmaceutical preparations, and concentrated the distribution in special organs and tissues in the body, and is targeting, thereby enhancing the efficacy of pharmaceutical preparations; and (4) compared with a liposome in the form of liquid, the liposome according to the present application is solid, has significantly improved stability and better reproducibility, and can be easily reconstituted, stored and transported.

The liposome according to the present application may be an ordinary liposome, a long circulating liposome, a thermosensitive liposome, an immune liposome, or other liposomes having special functions.

The liposome according to the present application may be administered to a patient or subject through any suitable route, such as, intravenous administration, intraarterial administration, intramuscular administration, intraperitoneal administration, subcutaneous administration, intraarticular administration, intrathecal administration, lateral intracerebroventricular administration, nasal spray, pulmonary inhalation, oral administration or other suitable administration routes known to those skilled in the art. The tissue lesions that can be treated with the liposome according to the present application include, but are not limited to tissue lesions from bladder, liver, lung, kidney, bone, soft tissue, muscle, breast, and the like.

### BRIEF DESCRIPTION OF THE DRAWINGS

Fig. 1 is a graph showing the tissue distribution of moexitecan in vivo after intravenous injection of 30mg/kg moexitecan liposome into a rat.
Fig. 2 is a graph showing the tissue distribution of an active metabolite SN38 in vivo after intravenous injection of 30mg/kg moexitecan liposome into a rat.
Fig. 3 is a graph showing the distribution of a fluorescent label IR623 and an IR623-labelled moexitecan liposome in a mouse; and
Fig. 4 is a graph showing the distribution of a fluorescent label IR623 and an IR623-labelled moexitecan liposome in a tumor site and each visceral organ in a mouse.

### EXAMPLES

The specific preparation methods according to the present application are illustrated by the following examples, but the protection scope of the present application is not limited thereto.

### Example 1

Formula:

| 1 kg of formulation amount | Formulated amount |
|---|---|
| Moexitecan | 2g |
| Yolk phosphatidylcholine | 30g |
| Hydrogenated soybean phosphatidylcholine | 10g |
| Anhydrous ethanol | 60g |
| Sucrose | 60g |
| Water for injection | Adding to 1000g |

Preparation process: formulated amounts of moexitecan, yolk phosphatidylcholine and hydrogenated soybean phosphatidylcholine were dissolved in a formulated amount of anhydrous ethanol under heating at 60 °C, and used as an organic phase; 70% of the formulation amount of water for injection was heated to 60 °C, and used as an aqueous phase; the organic phase was added to the aqueous phase upon shearing the aqueous phase to obtain a liposome feed liquid; the resulting liposome feed liquid was extruded through a 0.1 µm polycarbonate membrane 3 times; a formulated amount of sucrose was added; and then the resulting mixture was diluted to 1000g by adding water for injection again, subpackaged into vials for injection (penicillin bottle) made from neutral borosilicate glass tube, and then lyophilized to obtain liposomes. The entrapment efficiency measured by the ultrafiltration method was more than 99%.

### Example 2

Formula:

| 15kg of formulation amount | Formulated amount |
|---|---|
| Moexitecan | 30g |
| Yolk phosphatidylcholine | 450g |
| Hydrogenated soybean phosphatidylcholine | 150g |
| Anhydrous ethanol | 900g |
| Sucrose | 900g |
| Water for injection | Adding to 15kg |

Preparation process: formulated amounts of moexitecan, yolk phosphatidylcholine and hydrogenated soybean phosphatidylcholine were dissolved in a formulated amount of anhydrous ethanol under heating at 60 °C, and used as an organic phase; 70% of the formulation amount of water for injection was heated to 60 °C, and used as an aqueous phase; the organic phase was added to the aqueous phase upon shearing the aqueous phase to obtain a liposome feed liquid; the resulting liposome feed liquid was extruded through a 0.2 µm polycarbonate membrane 3 times; a formulated amount of sucrose was added; and then the resulting mixture was diluted to 15kg by adding water for injection again, subpackaged into vials for injection (penicillin bottles) made from neutral borosilicate glass tube, and then lyophilized to obtain liposomes. The entrapment efficiency measured by ultrafiltration method was more than 99%.

### Example 3

Formula:

| 1 kg of formulation amount | Formulated amount |
|---|---|
| Moexitecan | 2g |
| Yolk phosphatidylcholine | 30g |
| Hydrogenated soybean phosphatidylcholine | 10g |
| Anhydrous ethanol | 60g |
| Sucrose | 60g |
| Water for injection | Adding to 1000g |

Preparation process: formulated amounts of moexitecan, yolk phosphatidylcholine and hydrogenated soybean phosphatidylcholine were dissolved in a formulated amount of anhydrous ethanol under heating at 60 °C, and used as an organic phase; a formulated amount of sucrose was added to 70% of the formulation amount of water for injection and dissolved under heating at 60 °C to obtain a clear solution as an aqueous phase; the organic phase was added to the aqueous phase upon shearing or stirring the aqueous phase to obtain a liposome feed liquid; the resulting liposome feed liquid was extruded through a 0.4 µm polycarbonate membrane 3 times; and the extruded feed liquid was diluted to 1000g by adding water for injection again, subpackaged into vials for injection (penicillin bottles) made from neutral borosilicate glass tube, and then lyophilized to obtain liposomes. The entrapment efficiency measured by ultrafiltration method was more than 99%.

### Example 4

Formula:

| 1 kg of formulation amount | Formulated amount |
|---|---|
| Moexitecan | 2g |
| Yolk phosphatidylcholine | 30g |
| Hydrogenated soybean phosphatidylcholine | 10g |
| Anhydrous ethanol | 60g |
| Sucrose | 40g |
| Mannitol | 20g |
| Water for injection | Adding to 1000g |

Preparation process: formulated amounts of moexitecan, yolk phosphatidylcholine and hydrogenated soybean phosphatidylcholine were dissolved in a formulated amount of anhydrous ethanol under heating at 60 °C, and used as an organic phase; 70% of the formulation amount of water for injection was heated to 60 °C, and used as an aqueous phase; the organic phase was added to the aqueous phase upon shearing or stirring the aqueous phase to obtain a liposome feed liquid; the resulting liposome feed liquid was extruded through a 0.2 µm polycarbonate membrane 3 times; formulated amounts of sucrose and mannitol were added; and then the resulting mixture was diluted to 1000g by adding water for injection again, subpackaged into vials for injection (penicillin bottles) made from neutral borosilicate glass tube, and then lyophilized to obtain liposomes. The entrapment efficiency measured by ultrafiltration method was more than 99%.

### Example 5

Formula:

| 1 kg of formulation amount | Formulated amount |
|---|---|
| Moexitecan | 2g |
| Yolk phosphatidylcholine | 30g |
| Hydrogenated soybean phosphatidylcholine | 10g |
| BHT | 0.1g |
| Anhydrous ethanol | 60g |
| Sucrose | 60g |
| Water for injection | Adding to 1000g |

Preparation process: formulated amounts of BHT and moexitecan were added to a formulated amount of anhydrous ethanol, and dissolved under heating at 60 °C to obtain a clear solution, then formulated amounts of yolk phosphatidylcholine and hydrogenated soybean phosphatidylcholine were added, and dissolved under heating at 60 °C to obtain a clear solution as an organic phase; 70% of the formulation amount of water for injection was heated to 60 °C, and used as an aqueous phase; the organic phase was added to the aqueous phase upon shearing or stirring the aqueous phase to obtain a liposome feed liquid; the resulting liposome feed liquid was extruded through a 0.2 µm polycarbonate membrane to obtain a liposome feed liquid having a certain particle size and a certain particle size distribution; a formulated amount of sucrose was added; and then the resulting mixture was diluted to 1000g by adding water for injection again, subpackaged into vials for injection (penicillin bottles) made from neutral borosilicate glass tube, and then lyophilized to obtain liposomes. The entrapment efficiency measured by ultrafiltration method was more than 99%.

### Example 6

Formula:

| 1 kg of formulation amount | Formulated amount |
|---|---|
| Moexitecan | 2g |
| Yolk phosphatidylcholine | 30g |
| Hydrogenated soybean phosphatidylcholine | 10g |
| Disodium edetate | 0.1g |
| Anhydrous ethanol | 60g |
| Sucrose | 60g |
| Water for injection | Adding to 1000g |

Preparation process: a formulated amount of moexitecan was added to a formulated amount of anhydrous ethanol, and dissolved under heating at 60 °C to obtain a clear solution; then formulated amounts of yolk phosphatidylcholine and hydrogenated soybean phosphatidylcholine were added, and dissolved under heating at 60 °C to obtain a clear solution as an organic phase; a formulated amount of disodium edetate was dissolved in 70% of the formulation amount of water for injection under heating at 60 °C, and used as an aqueous phase; the organic phase was added to the aqueous phase upon shearing or stirring the aqueous phase to obtain a liposome feed liquid; the resulting liposome feed liquid was extruded through a 0.1 µm polycarbonate membrane to obtain a liposome feed liquid having a certain particle size and a certain particle size distribution; a formulated amount of sucrose was added; and then the resulting mixture was diluted to 1000g by adding water for injection again, subpackaged into vials for injection (penicillin bottles) made from neutral borosilicate glass tube, and then lyophilized to obtain liposomes. The entrapment efficiency measured by ultrafiltration method was more than 99%.

### Example 7

Formula:

| 1 kg of formulation amount | Formulated amount |
|---|---|
| Moexitecan | 2g |
| Yolk phosphatidylcholine | 30g |
| Hydrogenated soybean phosphatidylcholine | 10g |
| Hydrochloric acid or sodium hydroxide | Appropriate amount |
| Anhydrous ethanol | 60g |
| Sucrose | 60g |
| Water for injection | Adding to 1000g |

Preparation process: a formulated amount of moexitecan was fully dissolved in a formulated amount of anhydrous ethanol under heating at 60 °C to obtain a clear solution; formulated amounts of yolk phosphatidylcholine and hydrogenated soybean phosphatidylcholine were added and fully dissolved under heating at 60 °C to obtain a clear solution as an organic phase; 70% of the formulation amount of water for injection was kept warm at 60 °C, and used as an aqueous phase; the organic phase was added to the aqueous phase upon shearing or stirring the aqueous phase to obtain a liposome feed liquid; the resulting liposome feed liquid was extruded through a 0.2 µm polycarbonate membrane to obtain a liposome feed liquid having a certain particle size and a certain particle size distribution; a formulated amount of sucrose was added; hydrochloric acid or sodium hydroxide was added to adjust the pH to 5; and then the resulting mixture was diluted to 1000g by adding water for injection again, subpackaged into vials for injection (penicillin bottles) made from neutral borosilicate glass tube, and then lyophilized to obtain liposomes. The entrapment efficiency measured by ultrafiltration method was more than 99%.

### Example 8

Formula:

| 1 kg of formulation amount | Formulated amount |
|---|---|
| Moexitecan | 2g |
| Yolk phosphatidylcholine | 30g |
| Hydrogenated soybean phosphatidylcholine | 10g |
| Anhydrous ethanol | 60g |
| Sucrose | 60g |
| Water for injection | Adding to 1000g |

Preparation process: a formulated amount of moexitecan was fully dissolved in a formulated amount of anhydrous ethanol under heating at 60 °C to obtain a clear solution; formulated amounts of yolk phosphatidylcholine and hydrogenated soybean phosphatidylcholine were added and fully dissolved under heating at 60 °C to obtain a clear solution as an organic phase; a formulated amount of sucrose was dissolved in 70% of the formulation amount of water for injection under heating at 60 °C to obtain a solution as an aqueous phase; the organic phase was added to the aqueous phase upon shearing or stirring the aqueous phase to obtain a liposome feed liquid; the resulting liposome feed liquid was extruded through a 0.1 µm polycarbonate membrane to obtain a liposome feed liquid having a certain particle size and a certain particle size distribution; the extruded feed liquid was diluted to 1000g by adding water for injection again, subpackaged into vials for injection (penicillin bottles) made from neutral borosilicate glass tube, and then lyophilized to obtain liposomes. The entrapment efficiency measured by ultrafiltration method was more than 99%.

### Example 9 - Comparative

Formula:

| 1 kg of formulation amount | Formulated amount |
|---|---|
| Moexitecan | 2g |
| Yolk phosphatidylcholine | 30g |
| Hydrogenated yolk phosphatidylcholine | 10g |
| Anhydrous ethanol | 60g |
| Sucrose | 60g |
| Water for injection | Adding to 1000g |

Preparation process: a formulated amount of moexitecan was fully dissolved in a formulated amount of anhydrous ethanol under heating at 60 °C to obtain a clear solution; formulated amounts of yolk phosphatidylcholine and hydrogenated yolk phosphatidylcholine were added and fully dissolved under heating at 60 °C to obtain a clear solution as an organic phase; 70% of the formulation amount of water for injection was kept warm at 60 °C, and used as an aqueous phase; the organic phase was added to the aqueous phase upon shearing or stirring the aqueous phase to obtain a liposome feed liquid; the resulting liposome feed liquid was extruded through a 0.1 µm polycarbonate membrane to obtain a liposome feed liquid having a certain particle size and a certain particle size distribution; a formulated amount of sucrose was added; and then the resulting mixture was diluted to 1000g by adding water for injection again, subpackaged into vials for injection (penicillin bottles) made from neutral borosilicate glass tube, and then lyophilized to obtain liposomes. The entrapment efficiency measured by ultrafiltration method was more than 99%.

### Example 10

Formula:

| 1 kg of formulation amount | Formulated amount |
|---|---|
| Moexitecan | 2g |
| Yolk phosphatidylcholine | 22.5g |
| Hydrogenated soybean phosphatidylcholine | 7.5g |
| Anhydrous ethanol | 60g |
| Sucrose | 60g |
| Water for injection | Adding to 1000g |

Preparation process: specific operation steps are identical to those in Example 2. The entrapment efficiency measured by ultrafiltration method was more than 99%.

### Example 11

Formula:

| 1 kg of formulation amount | Formulated amount |
|---|---|
| Moexitecan | 2g |
| Yolk phosphatidylcholine | 150g |
| Hydrogenated soybean phosphatidylcholine | 50g |
| Anhydrous ethanol | 60g |
| Sucrose | 60g |
| Water for injection | Adding to 1000g |

Preparation process: specific operation steps are identical to those in Example 2. The entrapment efficiency measured by ultrafiltration method was more than 99%.

### Example 12

Formula:

| 1 kg of formulation amount | Formulated amount |
|---|---|
| Moexitecan | 2g |
| Yolk phosphatidylcholine | 30g |
| Hydrogenated soybean phosphatidylcholine | 10g |
| Anhydrous ethanol | 18g |
| Sucrose | 60g |
| Water for injection | Adding to 1000g |

Preparation process: specific operation steps are identical to those in Example 2. The entrapment efficiency measured by ultrafiltration method was more than 99%.

### Example 13

Formula:

| 1 kg of formulation amount | Formulated amount |
|---|---|
| Moexitecan | 2g |
| Yolk phosphatidylcholine | 30g |
| Hydrogenated soybean phosphatidylcholine | 10g |
| Anhydrous ethanol | 100g |
| Sucrose | 60g |
| Water for injection | Adding to 1000g |

Preparation process: specific operation steps are identical to those in Example 2. The entrapment efficiency measured by ultrafiltration method was more than 99%.

### Example 14 - Comparative

Formula:

| 1 kg of formulation amount | Formulated amount |
|---|---|
| Paclitaxel | 2g |
| Yolk phosphatidylcholine | 30g |
| Hydrogenated soybean phosphatidylcholine | 10g |
| Anhydrous ethanol | 60g |
| Sucrose | 60g |
| Water for injection | Adding to 1000g |

Note: except that moexitecan in the formula was replaced with paclitaxel, each formula and each process in Examples 2-13 were also applicable to this comparative example.

Preparation process: formulated amounts of paclitaxel, yolk phosphatidylcholine and hydrogenated soybean phosphatidylcholine were dissolved in a formulated amount of anhydrous ethanol under heating at 60 °C, and used as an organic phase; 70% of the formulation amount of water for injection was heated to 60 °C, and used as an aqueous phase; the organic phase was added to the aqueous phase upon shearing the aqueous phase to obtain a liposome feed liquid; the resulting liposome feed liquid was extruded through a 0.1 µm polycarbonate membrane 3 times; a formulated amount of sucrose was added, and then the resulting mixture was diluted to 1000g by adding water for injection again, subpackaged into vials for injection (penicillin bottles) made from neutral borosilicate glass tube, and then lyophilized to obtain liposomes. The entrapment efficiency measured by ultrafiltration method was more than 99%.

### Example 15 - Comparative

Formula:

| 1 kg of formulation amount | Formulated amount |
|---|---|
| Docetaxel | 2g |
| Yolk phosphatidylcholine | 30g |
| Hydrogenated soybean phosphatidylcholine | 10g |
| Anhydrous ethanol | 60g |
| Sucrose | 60g |
| Water for injection | Adding to 1000g |

Note: except that moexitecan in the formula was replaced with docetaxel, each formula and each process in Examples 2-13 were also applicable to this comparative example.

Preparation process: formulated amounts of docetaxel, yolk phosphatidylcholine and hydrogenated soybean phosphatidylcholine were dissolved in a formulated amount of anhydrous ethanol under heating at 60 °C, and used as an organic phase; 70% of the formulation amount of water for injection was heated to 60 °C, and used as an aqueous phase; the organic phase was added to the aqueous phase upon shearing the aqueous phase to obtain a liposome feed liquid; the resulting liposome feed liquid was extruded through a 0.1 µm polycarbonate membrane 3 times; a formulated amount of sucrose was added; and then the resulting mixture was diluted to 1000g by adding water for injection again, subpackaged into vials for injection (penicillin bottles) made from neutral borosilicate glass tube, and then lyophilized to obtain liposomes. The entrapment efficiency measured by ultrafiltration method was more than 99%.

### Example 16 - Comparative

Formula:

| 1 kg of formulation amount | Formulated amount |
|---|---|
| Tacrolimus | 2g |
| Yolk phosphatidylcholine | 30g |
| Hydrogenated soybean phosphatidylcholine | 10g |
| Anhydrous ethanol | 60g |
| Sucrose | 60g |
| Water for injection | Adding to 1000g |

Note: except that moexitecan in the formula was replaced with tacrolimus, each formula and each process in Examples 2-13 were also applicable to this comparative example.

Preparation process: formulated amounts of tacrolimus, yolk phosphatidylcholine and hydrogenated soybean phosphatidylcholine were dissolved in a formulated amount of anhydrous ethanol under heating at 60 °C, and used as an organic phase; 70% of the formulation amount of water for injection was heated to 60 °C, and used as an aqueous phase; the organic phase was added to the aqueous phase upon shearing the aqueous phase to obtain a liposome feed liquid; the resulting liposome feed liquid was extruded through a 0.1 µm polycarbonate membrane 3 times; a formulated amount of sucrose was added; and then the resulting mixture was diluted to 1000g by adding water for injection again, subpackaged into vials for injection (penicillin bottles) made from neutral borosilicate glass tube, and then lyophilized to obtain liposomes. The entrapment efficiency measured by ultrafiltration method was more than 99%.

### Example 17 - Comparative

Formula:

| 1 kg of formulation amount | Formulated amount |
|---|---|
| Alprazolam | 2g |
| Yolk phosphatidylcholine | 30g |
| Hydrogenated soybean phosphatidylcholine | 10g |
| Anhydrous ethanol | 60g |
| Sucrose | 60g |
| Water for injection | Adding to 1000g |

Note: except that moexitecan in the formula was replaced with alprazolam, each formula and each process in Examples 2-13 were also applicable to this comparative example.

Preparation process: formulated amounts of alprazolam, yolk phosphatidylcholine and hydrogenated soybean phosphatidylcholine were dissolved in a formulated amount of anhydrous ethanol under heating at 60 °C, and used as an organic phase; 70% of the formulation amount of water for injection was heated to 60 °C, and used as an aqueous phase; the organic phase was added to the aqueous phase upon shearing the aqueous phase to obtain a liposome feed liquid; the resulting liposome feed liquid was extruded through a 0.1 µm polycarbonate membrane 3 times; a formulated amount of sucrose was added; then the resulting mixture was diluted to 1000g by adding water for injection again, subpackaged into vials for injection (penicillin bottles) made from neutral borosilicate glass tube, and then lyophilized to obtain liposomes. The entrapment efficiency measured by ultrafiltration method was more than 99%.

### Comparison Example 1: Film Dispersion Method

Formula:

| 100g of formulation amount | Formulated amount |
|---|---|
| Moexitecan | 0.2g |
| Yolk phosphatidylcholine | 3g |
| Hydrogenated soybean phosphatidylcholine | 1g |
| Anhydrous ethanol | 6g |
| Sucrose | 6g |
| Water for injection | Adding to 100g |

Preparation process: formulated amounts of moexitecan, yolk phosphatidylcholine and hydrogenated soybean phosphatidylcholine were dissolved in a formulated amount of anhydrous ethanol under heating at 60 °C, and used as an organic phase; the organic phase was placed in a pear-shaped flask, which was then placed in a rotary evaporator, and subsequently rotary-evaporated at 60 °C under reduced pressure to removed ethanol, so that the organic phase formed a thin film; 70% of the formulation amount of water for injection was heated to 60 °C, and used as an aqueous phase; the aqueous phase was added to the flask having the thin film formed by rotary evaporation to form a liposome feed liquid after hydration; the resulting liposome feed liquid was extruded through a 0.2 µm polycarbonate membrane; a formulated amount of sucrose was added; and then the resulting mixture was diluted to 100g by adding water for injection again, subpackaged into vials for injection (penicillin bottles) made from neutral borosilicate glass tube, and then lyophilized to obtain liposomes.

Results: hydration cannot be carried out smoothly, and it is difficult to form a homogenous liposome feed liquid. Furthermore, the resulting liposome feed liquid cannot be extruded through the polycarbonate membrane, and settled and layered after being left to stand. Therefore, the film dispersion method was not suitable for preparing liposomes of moexitecan.

### Comparison Example 2: Micro-jet Homogenization Method

Formula:

| 1 kg of formulation amount | Formulated amount |
|---|---|
| Moexitecan | 2g |
| Yolk phosphatidylcholine | 30g |
| Hydrogenated soybean phosphatidylcholine | 10g |
| Anhydrous ethanol | 60g |
| Sucrose | 60g |
| Water for injection | Adding to 1000g |

Preparation process: formulated amounts of moexitecan, yolk phosphatidylcholine and hydrogenated soybean phosphatidylcholine were dissolved in a formulated amount of anhydrous ethanol under heating at 60 °C, and used as an organic phase; 70% of the formulation amount of water for injection was heated to 60 °C, and used as an aqueous phase; the organic phase was added to the aqueous phase upon shearing the aqueous phase to obtain a liposome feed liquid; the resulting liposome feed liquid was homogenized by micro-jet; a formulated amount of sucrose was added; and then the resulting mixture was diluted to 1000g by adding water for injection again, subpackaged into vials for injection (penicillin bottles) made from neutral borosilicate glass tube, and then lyophilized to obtain liposomes.

Results: the resulting liposome sample was difficultly reconstituted, and the reconstituted liposomes had very large particle size and very wide particle size distribution.

### Comparison Example 3: Micelle Preparation 1

| 1 kg of formulation amount | Formulated amount |
|---|---|
| Moexitecan | 2g |
| Cremophor | 30g |
| Glycerol | 10g |
| Anhydrous ethanol | 58g |

Preparation process: formulated amounts of moexitecan, cremophor and glycerol were fully dissolved in a formulated amount of anhydrous ethanol under heating at 45 °C in a water bath to obtain a clear solution; and the resulting clear solution was sterilized by filtration, and then subpackaged to obtain the micelle preparation.

### Comparison Example 4: Emulsion

| 1 kg of formulation amount | Formulated amount |
|---|---|
| Moexitecan | 1g |
| Vitamin E | 50g |
| F68(poloxamer 188) | 20g |
| Water | 1000 |
| Anhydrous ethanol | 10g |

Preparation process: ① formulated amounts of moexitecan and vitamin E were fully dissolved in a formulated amount of anhydrous ethanol under heating to obtain a clear solution; ② a formulated amount of F68 was fully dissolved in a formulated amount of water to obtain a clear solution; ③ the solution obtained from ① was added to a half of the solution obtained from ② upon shearing the half of the solution obtained from ②, and after fully shearing, the other half of the solution obtained from ② was added thereto, and fully mixed under shearing; and ④ the solution obtained from ③ was homogenized under high pressure 10 times, and then subpackaged to obtain emulsion.

### Comparison Example 5: Micelle Preparation 2

| 1 kg of formulation amount | Formulated amount |
|---|---|
| Moexitecan | 5g |
| Adding tert-butanol to | 1000g |

Preparation process: a formulated amount of moexitecan was fully dissolved in a formulated amount of tert-butanol under heating to obtain a clear solution, and then the clear solution was sterilized by filtration, subpackaged, and then lyophilized to obtain moexitecan powders .

| 1 kg of formulation amount | Formulated amount |
|---|---|
| ELP (polyoxylethylene castor oil ether (35)) | 315g |
| Glycerol | 105g |
| Anhydrous ethanol (pharmaceutical grade) | 610g |

Preparation process: formulated amounts of ELP, glycerol and anhydrous ethanol were uniformly mixed, sterilized by filtration, and then subpackaged to obtain a special solvent.

Usage: the moexitecan powders were dissolved in a 100-fold amount of the special solvent to obtain an injection, which was diluted and then administered to a subject.

### Comparison Example 6: High-pressure Homogenization Method

Formula:

| 1 kg of formulation amount | Formulated amount |
|---|---|
| Moexitecan | 2g |
| Yolk phosphatidylcholine | 30g |
| Hydrogenated soybean phosphatidylcholine | 10g |
| Anhydrous ethanol | 60g |
| Sucrose | 60g |
| Water for injection | Adding to 1000g |

Preparation process: formulated amounts of moexitecan, yolk phosphatidylcholine and hydrogenated soybean phosphatidylcholine were dissolved in a formulated amount of anhydrous ethanol under heating at 60 °C, and used as an organic phase; 70% of the formulation amount of water for injection was heated to 60 °C, and used as an aqueous phase; the organic phase was added to the aqueous phase upon shearing the aqueous phase to obtain a liposome feed liquid; and the resulting liposome feed liquid was homogenized under high pressure. The homogenized samples still had a large particle size of more than 500 nm, and the high-pressure homogenizer was difficult to normally operate.

### Comparison Example 7:

Formula:

| 1 kg of formulation amount | Formulated amount |
|---|---|
| Moexitecan | 2g |
| Yolk phosphatidylcholine | 30g |
| Hydrogenated soybean phosphatidylcholine | 10g |
| Anhydrous ethanol | 60g |
| Sucrose | 60g |
| Water for injection | Adding to 1000g |

Preparation process: formulated amounts of moexitecan, yolk phosphatidylcholine and hydrogenated soybean phosphatidylcholine were dissolved in a formulated amount of anhydrous ethanol under heating at 60 °C, and used as an organic phase; 70% of the formulation amount of water for injection was heated to 60 °C, and used as an aqueous phase; the organic phase was added to the aqueous phase upon shearing the aqueous phase to obtain a liposome feed liquid; the resulting liposome feed liquid was extruded through a 0.1 µm polycarbonate membrane 3 times; a formulated amount of sucrose was added; and then the resulting mixture was diluted to 1000g by adding water for injection again, subpackaged into vials for injection (penicillin bottles) made from neutral borosilicate glass tube.

Results: The appearance of the sample was changed significantly after it was placed at 40 °C for 15 days. That is, a white emulsion became a yellow emulsion.

### Example 18: Stability Test

Ten batches of pharmaceutical compositions comprising moexitecan were prepared in accordance with the formulas and preparation processes in Examples 1-7 and Comparison Examples 3, 4, 5 and 7. Each batch of samples was stored at 40 °C for 15 days. The appearance of and related substances in the samples were detected, and compared with those on the 0th day. The results were shown as follows.

| Example | 0th day | | 40°C 15th day | |
|---|---|---|---|---|
| | Appearance | Total impurity (%) | Appearance | Total impurity (%) |
| Example 1 | White solid | 1.15 | White solid | 1.65 |
| Example 2 | White solid | 1.14 | White solid | 1.63 |
| Example 3 | White solid | 1.17 | White solid | 1.68 |
| Example 4 | White solid | 1.12 | White solid | 1.59 |
| Example 5 | White solid | 1.15 | White solid | 1.65 |
| Example 6 | White solid | 1.13 | White solid | 1.67 |
| Example 7 | White solid | 1.14 | White solid | 1.63 |
| Comparison Example 3 | Colorless clear liquid | 1.11 | Yellow liquid | 45.54 |
| Comparison Example 4 | White emulsion | 1.77 | Yellow liquid | 43.13 |
| Comparison Example 5 | White powder | 1.13 | White powder | 1.63 |
| Comparison Example 7 | White emulsion | 1.19 | Yellow liquid | 45.07 |

### Example 19: Long-term Stability Test

Seven batches of pharmaceutical compositions comprising moexitecan were prepared in accordance with the formulas and preparation processes in Examples 1-7. Each batch of pharmaceutical compositions was stored at 6°C and 25°C, respectively. The samples stored at 6 °C were taken at the 3rd, 6th, 9th and 12th months, respectively, to detect related substances, and the samples stored at 25 °C were taken at the 1st, 2nd, 3rd and 6th months, respectively, to detect related substances, both of which were compared with those on the 0th day. The results were shown as follows.

| Example | Related substances-total impurity (%) | | | | |
|---|---|---|---|---|---|
| | 0th month | 25°C 1st month | 25°C 2nd month | 25°C 3rd month | 25°C 6th month |
| Example 1 | 1.15 | 1.17 | 1.20 | 1.23 | 1.44 |
| Example 2 | 1.14 | 1.15 | 1.19 | 1.22 | 1.46 |
| Example 3 | 1.17 | 1.16 | 1.18 | 1.21 | 1.47 |
| Example 4 | 1.12 | 1.15 | 1.18 | 1.21 | 1.45 |
| Example 5 | 1.15 | 1.16 | 1.19 | 1.24 | 1.48 |
| Example 6 | 1.13 | 1.16 | 1.19 | 1.23 | 1.49 |
| Example 7 | 1.12 | 1.14 | 1.18 | 1.23 | 1.51 |
| | | | | | |

| Example | Related substances-total impurity (%) | | | | |
|---|---|---|---|---|---|
| | 0th month | 6°C 3rd month | 6°C 6th month | 6°C 9th month | 6°C 12th month |
| Example 1 | 1.15 | 1.15 | 1.14 | 1.16 | 1.24 |
| Example 2 | 1.14 | 1.15 | 1.15 | 1.16 | 1.21 |
| Example 3 | 1.17 | 1.16 | 1.17 | 1.18 | 1.20 |
| Example 4 | 1.12 | 1.13 | 1.11 | 1.16 | 1.21 |
| Example 5 | 1.15 | 1.15 | 1.14 | 1.19 | 1.22 |
| Example 6 | 1.13 | 1.13 | 1.14 | 1.20 | 1.24 |
| Example 7 | 1.13 | 1.12 | 1.14 | 1.19 | 1.22 |

### Example 20: Measurement of Particle Size

Ten batches of pharmaceutical compositions comprising moexitecan were prepared in accordance with the formulas and preparation processes in Examples 1-9 and Comparison Example 2. One vial of pharmaceutical composition for each batch was reconstituted in water, and then sampled to measure the particle size with a nanometer particle size analyzer. The results were shown as follows.

| Example | Particle size before lyophilization | | Particle size after lyophilization and reconstitution | |
|---|---|---|---|---|
| | Average particle size | Dispersion coefficient | Average particle size | Dispersion coefficient |
| Example 1 | 95.8 | 0.127 | 109.0 | 0.212 |
| Example 2 | 178.5 | 0.183 | 204.5 | 0.199 |
| Example 3 | 351.3 | 0.233 | 399.1 | 0.353 |
| Example 4 | 128.0 | 0.132 | 149.6 | 0.224 |
| Example 5 | 178.5 | 0.174 | 184.5 | 0.193 |
| Example 6 | 149.6 | 0.089 | 176.5 | 0.104 |
| Example 7 | 233.9 | 0.164 | 245.7 | 0.188 |
| Example 8 | 96.5 | 0.106 | 119.8 | 0.140 |
| Example 9 | 95.2 | 0.085 | 122.9 | 0.118 |
| Comparison Example 2 | 91.0 | 0.511 | 7998.1 | 2.022 |

### Example 21: Toxicity Text

One batch of pharmaceutical compositions comprising moexitecan was prepared in accordance with the formula and preparation process in Example 2, and one batch of micelle preparations comprising moexitecan was prepared in accordance with the formula and preparation process in Comparison Example 5. The resulting two batches of pharmaceutical preparations were subjected to acute toxicity test in mice, acute toxicity test in rats, and toxicity test in rats after administration for 4 weeks at the same dosage. The results of toxicity tests for the two pharmaceutical preparations were compared, and the results were shown as follows:

| Example | Acute toxicity in mice | Acute toxicity in rats | Toxicity in rats after administration for 4weeks |
|---|---|---|---|
| Example 2 | No significant abnormality after administration | No significant abnormality after administration | No significant abnormality after administration |
| | | Five days after administration, about 1% reduction in body weight | Rat mortality: 0% |
| | Mice mortality: 40% | Rat mortality: 0% | No significant abnormality inthe lungs after gross dissection |
| Comparison Example 5 | Significant abnormality after administration | Significant abnormality after administration | Significant abnormality after administration |
| | | Five days after administration, about 10% reduction in body weight | Rat mortality: 75% |
| | Mice mortality: 65% | | Significant abnormality in the lungs after gross dissection |
| | | Rat mortality: 30% | |

### Example 22: Pharmacodynamic Test

One batch of pharmaceutical compositions comprising moexitecan was prepared in accordance with the formula and preparation process in Example 2, and one batch of micelle preparations comprising moexitecan was prepared in accordance with the formula and preparation process in Comparison Example 5. The resulting two batches of pharmaceutical preparations were subjected to a pharmacodynamic test in nude mice with NCI-H292 lung cancer, i.e., inhibitory effect on xenograft tumor growth. Results of the pharmacodynamic test for the two pharmaceutical preparations were shown as follows.

| Example | Inhibitory effect on xenograft tumor growth in nude mice with NCI-H292 lung cancer |
|---|---|
| Example 2 | The pharmaceutical preparation obtained in Example 2 is superior to that obtained in Comparison Example 5 at the same dosage of 10 mg/kg. |
| Comparison Example 5 | |

### Example 23: Long-term Toxicity Test

One batch of pharmaceutical compositions comprising moexitecan was prepared in accordance with the formula and preparation process in Example 2, and one batch of micelle preparations comprising moexitecan was prepared in accordance with the formula and preparation process in Comparison Example 5. The resulting two batches of pharmaceutical preparations were subjected to a long-term toxicity test in rats at a dosage of 60, 30 or 10 mg/kg. The test results of the two pharmaceutical preparations were compared, and shown as follows.

| Example | Rats |
|---|---|
| Example 2 | Death: 3/6 deaths in the 60 mg/kg group, and no deaths in other groups |
| | Other symptoms: myelosuppression was found in each group, and showed dose-dependency |
| | The toxicity was reduced by about 5 times, compared with the toxicity of the pharmaceutical preparation obtained in Comparison Example 5. |
| Comparison Example 5 | Death: 6/6 deaths in the 60 mg/kg group, 5/6 deaths in the 30 mg/kg group, and no deaths in the 10 mg/kg group. |
| | Other symptoms: myelosuppression was found in each group, and showed dose-dependency |

### Example 24: Tissue Distribution

One batch of pharmaceutical compositions comprising moexitecan was prepared in accordance with the formula and preparation process in Example 2. 18 SD rats were divided into three groups with 6 rats (3 female ones and 3 male ones) in each group. Rats in each group were intravenously injected via tail vein with a pharmaceutical composition comprising moexitecan at a dosage of 30 mg/kg. The rats were anesthetized at 15 min, 2 h and 6 h after administration, and then taken blood samples and tissues. The blood samples and tissues were respectively treated to obtain blood plasma and tissue homogenate samples, and moexitecan and its active metabolite SN-38 (chemical name: 20(s)-7-ethyl-10-hydroxycamptothecine) in the blood plasma and tissue homogenate samples were determined using an LC-MS/MS method. The results were shown in Fig. 1 and Fig. 2. Fig. 1 showed that moexitecan was mainly distributed in the organs, such as the rectum, liver, lung, blood plasma, colon, kidney, ovary, and heart. Fig. 2 showed that SN-38 was mainly distributed in the organs, such as the colon, rectum, liver, lung, blood plasma, ovary, jejunum, ileum, duodenum, and kidney. The concentrations of moexitecan and SN-38 in the rectum are very high. The concentration of moexitecan in the colon is lower than that in the blood plasma, but the concentration of the active metabolite SN-38 of moexitecan in the colon is highest, indicating the concentrated distribution of the pharmaceutical composition according to the present application in a special organ or tissue. Moexitecan and SN-38 both had the lowest concentrations in the cerebrum and testis.

### Example 25: In vivo Targeting Research

A batch of pharmaceutical compositions (particle size: about 100 nm) comprising moexitecan and fluorescence probe (IR623) was prepared according to the formula (additionally adding about 0.8% (w/w, by weight of the total amount of phospholipids in the formula as 100%) DSPE conjugated with a fluorescence probe IR623 (added and dissolved in an organic phase)) and the preparation process in Example 1. A batch of pharmaceutical compositions (particle size: about 400 nm) comprising moexitecan and fluorescence probe (IR623) was prepared according to the formula (additionally adding about 0.8% (w/w, by weight of the total amount of phospholipids in the formula as 100%) DSPE conjugated with a fluorescence probe IR623 (added and dissolved in an organic phase)) and the preparation process in Example 3. The two batches of pharmaceutical compositions were used for an in vivo targeting research in nude mice bearing intestinal cancer HT29 using near-infrared in vivo imaging technique, and were compared with the in vivo targeting of the fluorescence probe IR623. The results were shown in Fig. 3 and Fig. 4.

Results: there were obvious fluorescence signals in the abdomen at 0.5 h after a free fluorescence probe was injected into mice via tail vein. The fluorescence signals gradually weakened over time, and were metabolized to the outside (results as shown in Fig. 3). For mice injected with moexitecan liposomes containing the fluorescence probe and having a particle size of about 100 nm, fluorescence signals spread throughout the body at 0.5 h, began to concentrate at a tumor site at 4 h, were strongest at the tumor site at 8 h, began to weaken at the tumor site after 8 h, and still were present at the tumor site at 48 h (results as shown in Fig. 3). For mice injected with moexitecan liposomes containing the fluorescence probe and having a particle size of about 400 nm, fluorescence signals were obvious in the abdomen at 0.5 h, enhanced in the abdomen at 4 h, and still concentrated in the abdomen thereafter (results as shown in Fig. 3).

The tumor-bearing mice were dissected at 48 h after drug injection. Each visceral organ (tumor, liver, spleen, kidney and intestine) in the body was excised, and the fluorescence distribution in each visceral organ was observed using an in-vivo imager. It can be seen from Fig. 4 that the fluorescence in the organs of mice injected with the free fluorescent probe was very weak, and almost completely metabolized. Among the organs of mice injected with moexitecan liposomes containing the fluorescence probe and having a particle size of about 100nm, the fluorescence in the tumor was stronger than that in other organs. Among the organs of mice injected with moexitecan liposomes containing the fluorescence probe and having a particle size of about 400nm, the fluorescence in the liver was strongest.

It therefore can be concluded that the moexitecan liposomes containing the fluorescence probe and having a particle size of about 100 nm had passive tumor targeting, and the moexitecan liposomes containing the fluorescence probe and having a particle size of about 400 were mainly accumulated in the liver. Fluorescence probes were excreted mainly through intestinal and renal metabolism.

## Claims

1. A method for preparing a liposome, comprising
(1) dissolving a substance to-be-entrapped and a phospholipid in an organic solvent to obtain an organic phase, and then mixing the organic phase with an aqueous phase to obtain a liposome feed liquid;
(2) extruding the liposome feed liquid obtained in step (1) through a polycarbonate membrane; and
(3) lyophilizing,
wherein the phospholipid is a combination of yolk phosphatidylcholine and hydrogenated soybean phosphatidylcholine; and
the substance to-be-entrapped is moexitecan.

2. The method according to claim 1, wherein a lyoprotectant is added to the aqueous phase in step (1) or before performing the lyophilization in step (3).

3. The method according to claim 2, wherein the step (3) further comprises adding water for injection, sterilizing by filtration, and subpackaging prior to lyophilizing;

4. The method according to any one of claims 1-3, wherein a weight ratio of yolk phosphatidylcholine to hydrogenated soybean phosphatidylcholine is 3:1.

5. The method according to claim 3, wherein the organic solvent in step (1) is one or more selected from the group consisting of anhydrous ethanol, 95% ethanol, methanol, propanol, tert-butanol, n-butanol, acetone, methylpyrrolidone, ethyl acetate, isopropyl ether, and diethyl ether; and preferably, the organic solvent is selected from the group consisting of anhydrous ethanol, 95% ethanol and tert-butanol.

6. The method according to any one of claims 1-5, wherein a weight ratio of the substance to-be-entrapped to the phospholipid in step (1) is 1:1-500; preferably 1:1-100; more preferably 1:15-50; and still more preferably 1:20.

7. The method according to any one of claims 1-6, wherein a weight ratio of the substance to-be-entrapped to the organic solvent in step (1) is 1:1-100; preferably 1:9-50; and more preferably 1:30.

8. The method according to any one of claims 1-7, wherein the aqueous phase comprises water as a major component or substantially consists of water, such as deionized water, distilled water, purified water, water for injection, and the like, and preferably water for injection.

9. The method according to any one of claims 1-8, wherein the aqueous phase further comprises a metal ion chelating agent, which is selected from the group consisting of disodium edetate, sodium calcium edetate, 1,2-diaminocyclohexane tetraacetic acid, diethylenetriamine pentaacetic acid, trisodium N-(2-hydroxyethyl)-ethylenediamine triacetate, and N-di(2-hydroxyethyl)glycine.

10. The method according to any one of claims 1-9, wherein the organic phase is mixed with the aqueous phase in step (1) at a temperature of 25-80°C, preferably 55-65°C.

11. The method according to any one of claims 1-10, wherein a pore size of the polycarbonate membrane is selected from the group consisting of 0.015, 0.03, 0.05, 0.08, 0.1, 0.2, 0.4, 0.6, 0.8, 1.0, 2.0, 3.0, 5.0, 8.0, 10.0, and 12.0 µm, preferably 0.1 µm or 0.2 µm.

12. The method according to any one of claims 1-11, wherein a temperature of the liposome feed liquid in step (2) is controlled at 25-80°C, preferably 55-65°C.

13. The method according to any one of claims 2-12, wherein the lyoprotectant is one or more selected from the group consisting of mannitol, glucose, galactose, sucrose, lactose, maltose, and mycose; preferably, the lyoprotectant is one or more selected from the group consisting of sucrose, mycose, and mannitol; more preferably, the lyoprotectant is selected from sucrose or a combination of sucrose and mannitol; and still more preferably, the lyoprotectant is selected from sucrose or a combination of sucrose and mannitol, wherein a weight ratio of sucrose to mannitol is 2:1.

14. The method according to any one of claims 1-13, wherein the organic phase in step (1) further comprises an antioxidant, which is one or more selected from the group consisting of sodium sulfite, sodium bisulfite, sodium pyrosulfite, sodium thiosulfate, vitamin C, ascorbyl palmitate, tert-butyl-4-hydroxyanisole, di-tert-butyl-4-hydroxytoluene, vitamin E acetate, cysteine, and methionine.

15. A liposome prepared by the method of any one of claims 1-14, wherein the liposome can be reconstituted after the addition of water or an aqueous solvent and the reconstituted liposome has a particle size of 50-400 nm or 100-250 nm, preferably wherein the liposome has an entrapment efficiency > 99% and a particle size distribution index of below 0.18.

## Patentansprüche

1. Verfahren zur Herstellung eines Liposoms, das Folgendes umfasst
(1) Lösen einer einzuschließenden Substanz und eines Phospholipids in einem organischen Lösungsmittel, um eine organische Phase zu erhalten, und anschließendes Mischen der organischen Phase mit einer wässrigen Phase, um eine Liposomen-Beschickungsflüssigkeit zu erhalten;
(2) Extrudieren der in Schritt (1) erhaltenen Liposomen-Beschickungsflüssigkeit durch eine Polycarbonatmembran; und
(3) Lyophilisieren,
wobei das Phospholipid eine Kombination aus Dotterphosphatidylcholin und hydriertem Sojabohnenphosphatidylcholin ist; und
die einzuschließende Substanz Moexitecan ist.

2. Verfahren nach Anspruch 1, wobei der wässrigen Phase in Schritt (1) oder vor der Durchführung der Gefriertrocknung in Schritt (3) ein Gefrierschutzmittel zugesetzt wird.

3. Verfahren nach Anspruch 2, wobei der Schritt (3) ferner die Zugabe von Wasser für Injektionszwecke, das Sterilisieren durch Filtration und das Unterverpacken vor dem Gefriertrocknen umfasst;

4. Verfahren nach einem der Ansprüche 1 bis 3, wobei das Gewichtsverhältnis von Dotterphosphatidylcholin zu hydriertem Sojabohnenphosphatidylcholin 3:1 beträgt.

5. Verfahren nach Anspruch 3, wobei das organische Lösungsmittel in Schritt (1) eines oder mehrere ist, die aus der Gruppe bestehend aus wasserfreiem Ethanol, 95%-igem Ethanol, Methanol, Propanol, tert-Butanol, n-Butanol, Aceton, Methylpyrrolidon, Ethylacetat, Isopropylether und Diethylether ausgewählt werden; und das organische Lösungsmittel vorzugsweise aus der Gruppe bestehend aus wasserfreiem Ethanol, 95%-igem Ethanol und tert-Butanol ausgewählt wird.

6. Verfahren nach einem der Ansprüche 1 bis 5, wobei das Gewichtsverhältnis der einzuschließenden Substanz zu dem Phospholipid in Schritt (1) 1:1 bis 500; vorzugsweise 1:1 bis 100; noch bevorzugter 1:15 bis 50; und noch bevorzugter 1:20 beträgt.

7. Verfahren nach einem der Ansprüche 1 bis 6, wobei das Gewichtsverhältnis der einzuschließenden Substanz zu dem organischen Lösungsmittel in Schritt (1) 1:1 bis 100, vorzugsweise 1:9 bis 50 und besonders bevorzugt 1:30 beträgt.

8. Verfahren nach einem der Ansprüche 1 bis 7, wobei die wässrige Phase Wasser als Hauptkomponente umfasst oder im Wesentlichen aus Wasser besteht, wie deionisiertes Wasser, destilliertes Wasser, gereinigtes Wasser, Wasser für Injektionszwecke und dergleichen, und vorzugsweise Wasser für Injektionszwecke.

9. Verfahren nach einem der Ansprüche 1 bis 8, wobei die wässrige Phase ferner einen Metallionen-Chelatbildner enthält, der aus der Gruppe ausgewählt wird, die aus Folgendem besteht:
Dinatriumedetat, Natriumcalciumedetat, 1,2-Diaminocyclohexantetraessigsäure,
Diethylentriaminpentaessigsäure, Trinatrium-N-(2-hydroxyethyl)-ethylendiamintriacetat und N-Di(2-hydroxyethyl)glycin.

10. Verfahren nach einem der Ansprüche 1 bis 9, wobei die organische Phase mit der wässrigen Phase in Schritt (1) bei einer Temperatur von 25 bis 80° C, vorzugsweise 55 bis 65° C, gemischt wird.

11. Verfahren nach einem der Ansprüche 1 bis 10, wobei eine Porengröße der Polycarbonatmembran ausgewählt wird aus der Gruppe bestehend aus 0,015, 0,03, 0,05, 0,08, 0,1, 0,2, 0,4, 0,6, 0,8, 1,0, 2,0, 3,0, 5,0, 8,0, 10,0 und 12,0 µm, vorzugsweise 0,1 µm oder 0,2 µm.

12. Verfahren nach einem der Ansprüche 1 bis 11, wobei die Temperatur der Liposomen-Beschickungsflüssigkeit in Schritt (2) auf 25 bis 80° C, vorzugsweise 55 bis 65° C, geregelt wird.

13. Verfahren nach einem der Ansprüche 2 bis 12, wobei das Lyoprotektivum eines oder mehrere ist, die aus der Gruppe bestehend aus Mannit, Glucose, Galactose, Saccharose, Lactose, Maltose und Mycose ausgewählt werden; wobei das Lyoprotektivum vorzugsweise eines oder mehrere ist, die aus der Gruppe ausgewählt werden, die aus Saccharose, Mycose und Mannitol besteht; noch bevorzugter wird das Lyoprotektivum aus Saccharose oder einer Kombination von Saccharose und Mannitol ausgewählt; und noch stärker bevorzugt wird das Lyoprotektivum aus Saccharose oder einer Kombination von Saccharose und Mannit ausgewählt, wobei ein Gewichtsverhältnis von Saccharose zu Mannitol 2:1 beträgt.

14. Verfahren nach einem der Ansprüche 1 bis 13, wobei die organische Phase in Schritt (1) ferner ein Antioxidationsmittel enthält, das eines oder mehrere ist, die aus der Gruppe bestehend aus Natriumsulfit, Natriumbisulfit, Natriumpyrosulfit, Natriumthiosulfat, Vitamin C, Ascorbylpalmitat, tert-Butyl-4-hydroxyanisol, Di-tert-Butyl-4-hydroxytoluol, Vitamin E-Acetat, Cystein und Methionin ausgewählt werden.

15. Liposom, das durch das Verfahren nach einem der Ansprüche 1 bis 14 hergestellt wird, wobei das Liposom nach Zugabe von Wasser oder einem wässrigen Lösungsmittel rekonstituiert werden kann und das rekonstituierte Liposom eine Teilchengröße von 50 bis 400 nm oder 100 bis 250 nm aufweist, vorzugsweise, wobei das Liposom eine Einschlusseffizienz > 99 % und einen Partikelgrößenverteilungsindex von unter 0,18 aufweist.

## Revendications

1. Procédé de préparation d'un liposome, comprenant
(1) la dissolution d'une substance devant être piégée et un phospholipide dans un solvant organique pour obtenir une phase organique, puis le mélange de la phase organique avec une phase aqueuse pour obtenir un liquide d'alimentation en liposomes ;
(2) l'extrusion du liquide d'alimentation en liposomes obtenu à l'étape (1) à travers une membrane en polycarbonate ; et
(3) la lyophilisation,
dans lequel le phospholipide est une combinaison de phosphatidylcholine de jaune et de phosphatidylcholine de soja hydrogénée ; et
la substance devant être piégée est le moexitecan.

2. Procédé selon la revendication 1, dans lequel un lyoprotecteur est ajouté à la phase aqueuse à l'étape (1) ou avant d'effectuer la lyophilisation à l'étape (3).

3. Procédé selon la revendication 2, dans lequel l'étape (3) comprend en outre l'ajout d'eau pour injection, la stérilisation par filtration et le sous-conditionnement avant la lyophilisation.

4. Procédé selon l'une quelconque des revendications 1 à 3, dans lequel un rapport pondéral de la phosphatidylcholine de jaune par rapport à la phosphatidylcholine de soja hydrogénée est de 3:1.

5. Procédé selon la revendication 3, dans lequel le solvant organique de l'étape (1) est un ou plusieurs choisis dans le groupe constitué par l'éthanol anhydre, l'éthanol à 95%, le méthanol, le propanol, le tert-butanol, le n-butanol, l'acétone, la méthylpyrrolidone, l'acétate d'éthyle, l'éther isopropylique et l'éther diéthylique ; et préférablement, le solvant organique est choisi dans le groupe constitué par l'éthanol anhydre, l'éthanol à 95% et le tert-butanol.

6. Procédé selon l'une quelconque des revendications 1 à 5, dans lequel un rapport pondéral de la substance devant être piégée par rapport au phospholipide à l'étape (1) est de 1:1-500 ; préférablement 1:1-100 ; plus préférablement 1:15-50 ; et encore plus préférablement 1:20.

7. Procédé selon l'une quelconque des revendications 1 à 6, dans lequel un rapport pondéral de la substance devant être piégée au solvant organique dans l'étape (1) est de 1:1-100 ; préférablement 1:9-50 ; et plus préférablement 1:30.

8. Procédé selon l'une quelconque des revendications 1 à 7, dans lequel la phase aqueuse comprend de l'eau en tant que composant majeur ou est composée essentiellement de l'eau, telle que de l'eau désionisée, de l'eau distillée, de l'eau purifiée, de l'eau pour injection, et similaires, et préférablement de l'eau pour injection.

9. Procédé selon l'une quelconque des revendications 1 à 8, dans lequel la phase aqueuse comprend en outre un agent chélatant d'ions métalliques, qui est choisi dans le groupe constitué par l'édétate disodique, l'édétate de sodium et de calcium, l'acide tétraacétique 1,2-diaminocyclonexane, l'acide pentaacétique diéthylènetriamine, le triacétate de N-(2-hydroxyéthyl)-éthylènediamine trisodique et le N-di(2-hydroxyéthyl) glycine.

10. Procédé selon l'une quelconque des revendications 1 à 9, dans lequel la phase organique est mélangée avec la phase aqueuse dans l'étape (1) à une température de 25 à 80°C, préférablement de 55 à 65°C.

11. Procédé selon l'une quelconque des revendications 1 à 10, dans lequel une taille de pore de la membrane en polycarbonate est choisie dans le groupe constitué de 0,015, 0,03, 0,05, 0,08, 0,1, 0,2, 0,4, 0,6, 0,8, 1,0, 2,0, 3,0, 5,0, 8,0, 10,0 et 12,0 µm, préférablement 0,1 µm ou 0,2 µm.

12. Procédé selon l'une quelconque des revendications 1 à 11, dans lequel une température du liquide d'alimentation en liposomes dans l'étape (2) est contrôlée à 25 à 80°C, préférablement 55 à 65°C.

13. Procédé selon l'une quelconque des revendications 2 à 12, dans lequel le lyoprotecteur est un ou plusieurs choisis dans le groupe constitué par le mannitol, le glucose, le galactose, le saccharose, le lactose, le maltose et le mycose ; préférablement, le lyoprotecteur est un ou plusieurs choisis dans le groupe constitué par le saccharose, le mycose et le mannitol ; plus préférablement, le lyoprotecteur est choisi parmi le saccharose ou une combinaison de saccharose et de mannitol ; et encore plus préférablement, le lyoprotecteur est choisi parmi le saccharose ou une combinaison de saccharose et de mannitol, dans lequel un rapport pondéral de saccharose par rapport au mannitol est de 2:1.

14. Procédé selon l'une quelconque des revendications 1 à 13, dans lequel la phase organique de l'étape (1) comprend en outre un antioxydant, qui est un ou plusieurs choisis dans le groupe constitué par le sulfite de sodium, le bisulfite de sodium, le pyrosulfite de sodium, le thiosulfate de sodium, la vitamine C, la palmitate d'ascorbyle, le tert-butyl-4-hydroxyanisole, le di-tert-butyl-4-hydroxytoluène, la vitamine E, l'acétate, la cystéine et la méthionine.

15. Liposome préparé par le procédé de l'une quelconque des revendications 1 à 14, dans lequel le liposome peut être reconstitué après l'ajout d'eau ou d'un solvant aqueux et le liposome reconstitué a une taille de particule de 50 à 400 nm ou de 100 à 250 nm, préférablement dans lequel le liposome a une efficacité de piégeage > 99 % et un indice de distribution granulométrique inférieur à 0,18.
